# EUROPEAN PATENT APPLICATION

(11) **EP 3 561 486 A1**
(43) Date of publication of application: **30.10.2019**
(21) Application number: 18020174.1
(22) Date of filing: 27.04.2018
(51) Int. Cl.: G01N 21/31, G01N 21/359, G01N 21/01

(54) **PORTATIVE OPTICAL SYSTEM FOR DETECTION OF CHEMICAL SUBSTANCES AT TRACE LEVELS IN FOODS AND LIQUIDS**

(71) Applicant: CERAGOS Electronics et Nature, 06800 Cagnes-Sur-Mer (FR)
(72) Inventor: Cerasani, Ennio, 06800 Cagnes-Sur-Mer (FR); Cerasani, Umberto, 06800 Cagnes-Sur-Mer (FR)

(57) **Abstract**

Foods and drinks adulterants present at trace levels constitute a major threat to human health. Optical spectroscopy has emerged as a promising method to discriminate micro-trace chemicals from naturally occurring molecules in foods and drinks, although often requiring addition of a chemical reagent. A non-invasive and portative trace chemical detection system is described, which comprises a light source (10, 11), a non-volatile memory element to store reference chemical spectral data, a computational unit (44) configured to perform an element-by-element division and a baseline filter. A trace chemical detection method is also disclosed using data server (54) communication to update and add reference chemical spectral data, in order to increase the number of micro-trace chemicals probed.

## Description

### Technical Field

The invention relates to the field of chemical traces analysis and more particularly to the technical field of chemical traces analysis in foods and liquids using a spectrophotometry detection system.

### Background Art

In recent years, foods and drinks poisoning caused by various types of hazardous substances and chemical elements with high atomic weight such as pesticides or heavy metals have been a major threat to human health. The current methods used to estimate chemical substances at trace levels for soil, plants, foods or water comprise gas chromatography, high-performance liquid chromatography, gas chromatography-mass spectroscopy, liquid chromatography-mass spectroscopy and other large equipment. Although these methods can maintain accuracy and precision of the data, they are often complicate to operate, time consuming and require professional assistance.

In recent years, biosensors have been developed to detect chemical residues at trace levels based on the principle of electrical quantification of specific enzymatic variations [1]. However, biosensors still often suffer from lack of sensitivity and limited number of utilization, restricting their use.

On the other hand, optical spectroscopy has emerged as a promising method, capable of detecting micro traces of chemical substances in a non-invasive and non-destructive manner. Furthermore, the miniaturization of spectrophotometer allowed their integration into portable devices [2] [3].

Chemical substances at trace levels adulterate foods or water forming a chemical mixture. However, foods or drinks natural occurring molecules and chemical substances at trace levels may absorb light radiations at similar wavelength. In such a case, their respective absorbance adds together according to the Beer-Lamberts concentration's law. Because of their low concentration and their overlapping absorbance, chemical adulterants at trace levels are therefore very challenging to quantify. Several approaches can be adopted to discriminate trace adulterants from other naturally occurring molecules, with the most employed methods being chemical reaction and multi-wavelength measurements.

Addition of a chemical reagent, which specifically reacts with the trace substance to analyse, forms a highly absorbing compound with an absorption maximum at a different wavelength that the trace substance to detect. Therefore, there is no longer interference between the analysed sample and the highly absorbing compound formed.

[4] describes a starch staining system spectrophotometric quantitative analysis method, comprising the steps of adding FeCl3 solution and an appropriate amount of H2O2. [5] describes a method for detecting a trace quantity of copper in wastewater, comprising the steps of adding DDTC-Na into the wastewater and detecting the extract phase obtained through an ultraviolet spectrophotometer.

Common procedures of pesticides extraction, prior to spectrophotometry measures, comprise chemical solvent extraction [6] [7] or dye sensitization [8] [9].

On the other hand, computational techniques have also been developed to discriminate trace substances from naturally occurring molecules in light absorption measurements. The multi-wavelength or multicomponents analysis technique has seen a resurgence of interest over the last few years, probably because of the progress in data processing techniques. Multicomponent analysis algorithms still suffer of significant experimentation bias since they often require the analyst to input few parameters comprising: the number of components, measurement wavelengths and concentration values of the standards.

[10] describes a Multivariate Pattern Regression Analysis of the NIR reflection spectrum of seafood. In [11], The authors attempted to apply Partial Least Squares regression on NIR for the quantification of fatty acids, sterols and wax in 40 chemically characterized olive oil samples, from different origins. In [12], NIR spectroscopy was utilized in combination to a PLS algorithm and a wavelet-based calibration algorithm to quantify the degree of adulteration of durum wheat flour. A multivariate screening methodology to detect adulterants in various foods including hazelnuts and chickpeas was realized in [13].

### Summary of invention

In the view of the foregoing problems, it is an object of the invention to provide an optical system which detects chemical substances at trace levels without chemical pretreatment. It is another object of the invention to provide an optical system which automatically process data rapidly, without the use of complex computation procedures.

It is a third object of the invention to progressively increase the number of chemical substances that can be detected. Trace analysis of chemical substances present in foods or liquids is often restricted to chemical traces of substances already characterized. According to an embodiment of the invention, the chemical traces are stored in a distant database capable of being updated with supplementary chemical traces.

In a preferred embodiment of the present invention, the data processing is performed with digital means. In another embodiment, the creation of analog circuit implementing these digital functions is realized, leading to increased system time response. In such a case, the analog system can be easily adapted to various optical system outputs, rendering the present invention of broad scope.

Reliable trace chemical analysis of substances present in foods or liquids, often require professional assistance and complex detection procedures. The present invention can be implemented in portative devices, rendering the detection of chemicals at trace levels present in foods or liquids rapid and convenient.

To search for a particular chemical substance corruption in trace amounts, this particular chemical substance must be characterized first. In that intent, this particular chemical substance is added to a solution of known concentration and spectral measurement are performed using the same detection device than the one used on foods or liquid samples. The spectral measurements of this particular chemical substance are stored in a non-volatile memory and are named in this document as reference chemical spectral data or equivalently as reference chemical light spectrum. In addition, the expression chemical substances includes but is not limited to chemical compounds, chemical elements such as adulterants or pollutants present in foods or liquids that may constitute a threat to human health or essential human body trace elements such as nutriments.

An illustrative application of the present invention is the detection of pesticides in foods or drinks.

Performing repetitive spectral measurements on the solution containing this particular chemical substance from different angles and averaging the results, lessen the obtained spectrum dependence on various noise sources including spectrophotometer analog noises.

Measured light spectrum of a food or a liquid sample containing the trace chemical substance to quantify can be mathematically separated into two parts: (1) a first part representing the food or liquid sample naturally occurring molecules and (2) a second part representing the spectral trace of the chemical substance to quantify.

Performing an element-by-element division on all the analysed wavelengths of the measured light spectrum of the food or liquid sample by each of the trace chemical probed light spectrum stored in a non-volatile memory, mathematically results in the addition of: (1) an unknown term and (2) a constant term representing the concentration of the chemical substance in the sample analysed.

A baseline filter is then used to remove the unknown term from the element-by-element division result, since the trace chemical concentration is constant after element-by-element division, giving the relevance of the detailed method.

In another embodiment of the present invention, chemical substance concentration in the analysed food or liquid is assessed by applying a linear regression model following measured spectrum element-by-element division.

In another embodiment of the present invention, linear regression model is solved using the Least Square Regression Method. The application of a linear regression model to the food or liquid sample light spectrum corrupted by chemical substances at trace levels is performed following previous element-by-element division of the measured light spectrum by the memory stored chemical substance light spectrum.

Overlapping peaks discrimination comprising derivative computation or resolution enhancing procedures, permits to identify the particular wavelengths where the analysed chemical substances light spectra are specifically relevant. In a preferred embodiment, only these wavelengths are used to determine the concentration of the trace chemical substances corrupting in the sample.

In a preferred embodiment of the present invention, where the sample to analyse is solid or opaque, there is provided:
a light source arranged and disposed to apply broadband light to a sample. The light source includes one or all two of a light emitting diode or a lamp. The light source is selected to emit in one or all three of the UltraViolet frequencies, Visible frequencies or InfraRed frequencies.
an optical detection system configured to receive the reflected light produced by applying the broadband light to the sample. The optical detection system comprises a spectrophotometer element, capable of being integrated inside a portable device
a non-volatile memory element to store the reference chemicals light spectrum. The non-volatile memory includes a Flash memory or an EPROM memory. Reference chemical substances of known concentration are dissolved in a predetermined solution. Each solution is then analysed with the same optical system and the same light source than the one used to analyse the foods or liquid sample. Results are then stored in a non-volatile memory element.
a computational unit configured to perform basic mathematical operations, comprising element-by-element mathematical divisions. In a preferred embodiment the computational unit capable of performing the element-by-element division comprises a microprocessor or a FPGA.

In a preferred embodiment, the same computational unit is used to perform overlapping peaks discrimination including derivative computation or other resolution enhancing procedures.
a baseline removal filter, which can be digitally implemented. In a preferred embodiment, the overlapping peak discrimination and the baseline removal filter are both performed with a Savitzky-Golay smoothing and differentiation filter.

A preferred embodiment of the present invention, may further include:
a chargeable battery comprising a Lithium Ion or a Nickel Cadmium battery. The battery further allowing the system to be portative.
a communication element configured to transmit information with a smart device or a computer including all two of a wireless transmission element and a hard-wired transmission. The wireless communication element is a Bluetooth receiving and transmitting device. The hard-wired transmission is attached to the smart device or the computer through an USB interface.
a built-in display system, including an OLED monitor or a LCD monitor, configured to present the computational unit output directly or indirectly to the user.

In another embodiment of the present invention, where the sample to analyse is nearly transparent including water or liquids, there is provided:
the optical detection system is then configured to receive the transmitted light produced by applying the broadband light to the sample,
each trace chemical substance is dissolved in a nearly transparent solution at a known concentration, including pure water. Each solution is measured with the optical detection system configured to receive transmitted light from the solution. The transmitted light spectrum of each solution containing a trace chemical substance is stored in a non-volatile memory,
For each analysed wavelength, the transmitted light spectrum of the analysed sample is then divided by the transmitted light spectrum of each trace chemical substance dissolved in solution.

In another embodiment of the present invention, the trace chemical detection system is entirely realized with analog circuits. The element-by-element division is replaced by a modified Gilbert multiplicative cell, the peak overlapping discrimination is realized using a differentiator analog circuit and the baseline filter is realized using a Butterworth filter implemented in Sallen and Key topology.

In a preferred embodiment of the present invention, the light spectra of reference chemicals are stored in a distant data server housing a database element. The light spectra of reference chemicals stored in a distant data server are then transmitted to the non-volatile memory element before comparison with measured light spectrum of the analysed sample.

In another embodiment of the present invention, the analysed sample measured light spectrum is transmitted to a smart device including a communication element, a computational unit, a non-volatile memory and an user interface such as a display. Said smart device then connects with the data server to receive and store the reference chemicals light spectra contained in the data server.

In one embodiment, the comparison between the samples measured light spectra and the reference chemical substances light spectra is directly performed by the smart device computational unit. The comparison results are then directly displayed to the smart device user.

### Brief description of drawings

A preferred embodiment of the present invention will now be described, by way of illustrative and non-limiting examples, with reference to the accompanying drawings.
Fig. 1. is an enlarged perspective view of the subassembly of the system in contact with the sample to analyse. This subassembly includes a lighting circuit board which comprises 4 white Lights Emitting Diodes.
Fig. 2. A is a perspective view of the spectrophotometer circuit board and the UV-Vis embedded spectrophotometer according to a preferred embodiment of the present invention. Fig. 2. B is another (back) perspective view of the spectrophotometer circuit board and the UV-Vis embedded spectrophotometer according to a preferred embodiment of the present invention.
Fig 3. is an assembly drawing of the embedded spectrophotometer circuit board with the lighting circuit board.
Fig. 4 is an exploded view in perspective, showing the internal components of optical detector.
Fig. 5 is a diagram illustrating the portative trace chemical detection device connection with the data server housing the database according to an embodiment.

### Description of embodiments

While the present teachings are described in conjunction with the preferred embodiment and examples, it is not intended that the present teachings be limited to such embodiments. On the contrary, the present teachings encompass various alternatives and equivalents, as will be appreciated by those of skill in the art.

In a preferred embodiment of the present invention, the trace chemical detection system is realized with a portative UV-Vis spectrophotometer 23 and coherent light sources 11. The foods or liquid samples to analyze are directly placed on the aperture of trace chemical detection system case 48. The light emitted from the broadband coherent Light Emitting Diodes (LED) 11 is reflected over the surface of the sample to analyse and enters the light emitting module aperture 12 to reach the spectrophotometer input slit 24.

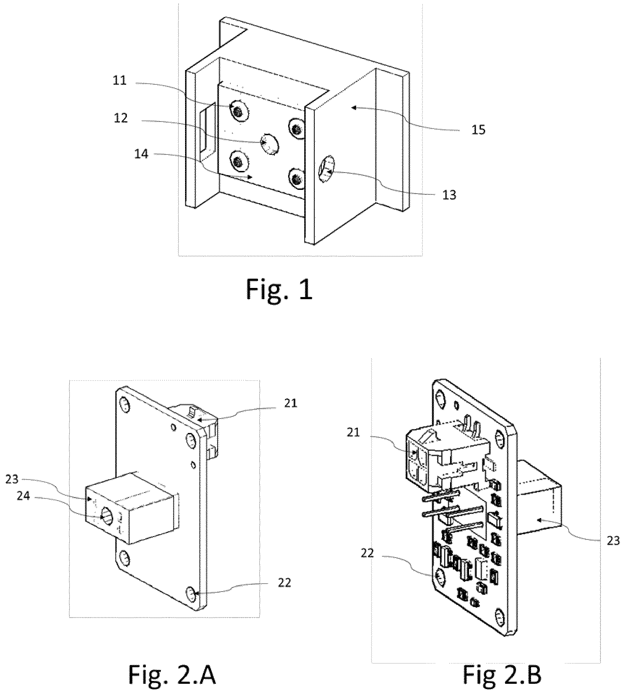

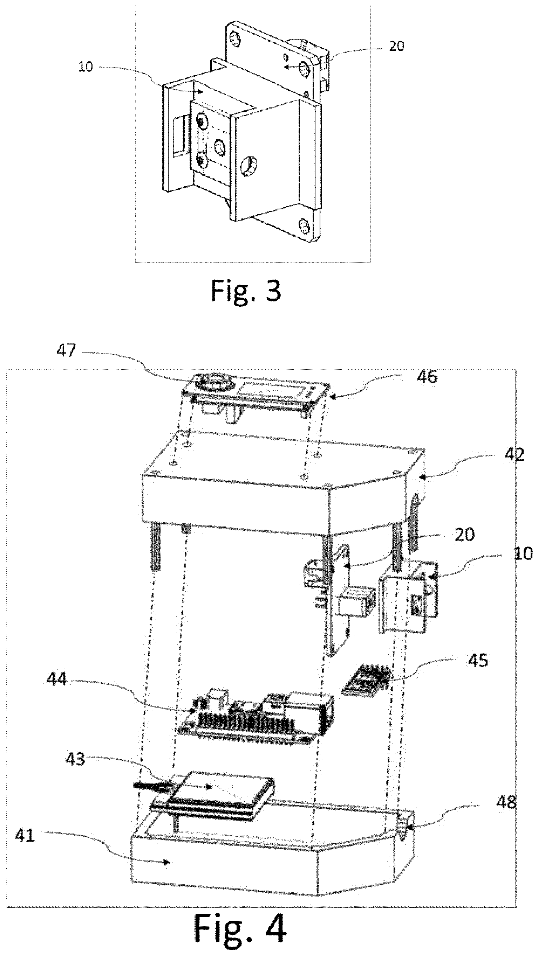

The light emitting module 10 is composed of the light emitting circuit board 14 and of the supporting receptacle 15. The supporting receptacle 15 is placed in front of the aperture of the detection system case 48, in way to align the light emitting module aperture 12 with the system case aperture 48. Light emitting circuit board 14 comprises 4 THORLAB white LEDs in SMT package in the embodiment presented in Fig. 1. Each LED lamp is tilted angularly such that the brightest light emission thereof is directed angularly toward the center of the system case aperture 48, where the sample to analyse is placed. Part of the LEDs reflected light enters the light emitting circuit board central aperture 12, where the spectrometer entrance slit 24 is positioned, according to the embodiment presented in Fig. 3. The light module supporting receptacle 15 is drilled on its sides 13 to allow LEDs power delivery through electrical leads.

In a preferred embodiment of the present invention, the optical detection system is a HAMAMATSU UV-Vis portative micro-spectrometer 23. The micro-spectrometer 23 is connected to an electrical driver board 20 configured to supply power, to control and to transfer data from the micro-spectrometer 23 to the system main control board 44. 4 fixation holes permit to secure the spectrometer driver board 20 to the system case 41.

According to the embodiment of Fig. 3, the light source module 20 is bond to the spectrometer driver board 20 with adhesive.

According to the embodiment of Fig.4, the system bottom case 41 is machined to receive various components, except the Liquid Crystal Display (LCD) 47. When the system is not connected to an energy source, the electric power is delivered from a 4.2V Lithium-Ion battery of 1Ah 43, which is fixed to the system bottom case 41 with adhesive.

According to the embodiment of Fig. 4, the system main control board 44 contains all two of the computational unit and the non-volatile memory. According to the embodiment of Fig. 4, the main control board is a STMicroelectronic Microcontroller integrating an ARM microprocessor and a Flash memory. The element-by-element division and the overlapping peak discrimination algorithms are executed by the ARM microprocessor. The chemical trace of chemical substance such as adulterants or pesticides are stored in the integrated Flash memory.

The reflection spectrum can also be pre-processed using other known statistical methods, e.g. a Standard Normal Variation (SNV) of the reflection spectrum can be computed before proceeding to element-by-element division. The slope and/or inflection of the spectral features in the reflection spectrum can be accounted for by performing Savitzky-Golay filtering of the reflection spectrum, and computing a first and/or second derivative of the reflection spectrum. Other statistical methods, such as sample-wise normalization and/or channel-wise auto-scaling of the reflection spectrum, can be used to facilitate noise reduction, and to provide more stable results.

According to the embodiment of Fig. 4, a Bluetooth transmission and reception module 45 is physically connected to the system main control board and is configured to transfer data with a distant smart device 52 including a computer or a smartphone.

The light source module 10 and the spectrometer driver board 20 assembly is positioned in such a way that the system case aperture 48 is in front of the light source circuit board aperture 12. The system case aperture 48 is configured to be placed close to the sample to analyse. The system case aperture 48 is of sufficient diameter and disposition to allow the LEDs rays of the light source circuit board 14 to be projected outside the assembled case and to reflect to the analysed sample. Part of the reflected light from the analysed sample penetrates inside the light source circuit board aperture 12 to reach the spectrometer entrance slit 24, for reflected light analysis.

The LCD display 46, containing the system command button 47, is fixed with screws on the system case top 42 and informs the operator about the computational results or other specific requirements.

Many variants of the optical detection system embodiment are clearly possible. For instance, the HAMAMATSU UV Vis micros-spectrophotometer can be replaced with a portable NIR micro-spectrophotometer and the white broadband LED can be replaced with IR emitting LEDs with glass lens.

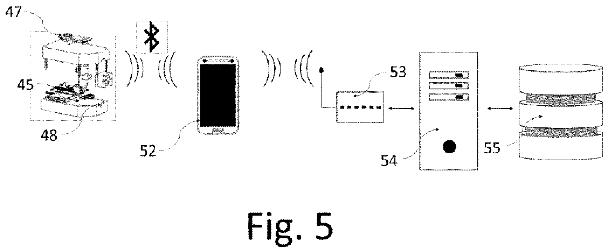

According to the embodiment of Fig. 5, measured foods and drinks light spectra are transmitted to an user smart device 52, using the Bluetooth communication element 45. The user smart device 52 is connected wirelessly to a data server 54 providing database 55 services. In a preferential embodiment, the data server 54 provides synchronization and offline capacities, ensuring that the database 55 update does not require network connection when the smart device 52 is offline or the optical detection system 23 is not working.

In another embodiment, a mobile application is installed on the user smart device 52 available from a distribution platform. Said mobile application is configured to transfer information from the data server 54 to the user smart device 52, to perform mathematical operations such as element-by-element division or derivation, to implement digital filters such as Savitzky-Golay filters and to display final results to the user smart device 52 using a user-friendly mobile interface.

### Citation list

[1] WO2007123967, (J. Zahner), 2006-04-20.
[2] US6043893A, (A. H. Treiman and T. D. Shelfer), 1998-10-09.
[3] CN202216899U, (C. Yu), 2011-09-20.
[4] CN103439279A, (Z. Xueyi, D. Dongli, W. Shuangyan, and Z. Zumin), 2013-09-10.
[5] CN104596967, (G. Jie, G. Yu, J. Meiling, C. Hui, and W. Qing), 2013-10-31.
[6] D. P. Condo and G. E. Janauer. Reactive pre-concentration of trace amounts of pesticides in environmental analysis procedures. Part I. Determination of Carbamates by indirect spectrophotometry. Analyst. 1987, vol. 112, p. 1027-1031.
[7] E.-A. B. Abd, M. R. Aleem, S. M. Khalile, and O. K. El-Naggar. Spectrophotometric Determination of Some Organophosphorus Pesticides Based on Reaction with Cerium (IV) Sulfate. 2016.
[8] H.-s. Liu and B.-j. Han. Determining General Capsaicin in Capsaicin-based Pesticides by Spectrophotometry. Modern Agrochemicals. 2008, vol. 7, p. 24.
[9] CN205941322U, (H. Liang), 2016-05-31.
[10] WO2014165331A1, (N. A. O'brien, C. A. Hulse, H. W. Siesler, and C. Hsiung), 2013-03-21.
[11] I. Delgadillo, A. Barros, and A. Nunes, "Quality evaluation of olives, olive pomace and olive oil by infrared spectroscopy," in Olive Oil-Constituents, Quality, Health Properties and Bioconversions, ed: InTech, 2012.
[12] M. Cocchi, C. Durante, G. Foca, A. Marchetti, L. Tassi, and A. Ulrici. Durum wheat adulteration detection by NIR spectroscopy multivariate calibration. Talanta. 2006, vol. 68, p. 1505-1511.
[13] M. I. López, E. Trullols, M. P. Callao, and I. Ruisánchez. Multivariate screening in food adulteration: untargeted versus targeted modelling. Food chemistry. 2014, vol. 147, p. 177-181

## Claims

1. A portable trace chemical detection system comprising: a light source arranged and disposed to apply broadband light to a sample, an optical detection system configured to receive the reflected light produced by applying the broadband light to the sample or configured to receive the transmitted light produced by applying the broadband light to the sample, a non-volatile memory element to store the light spectrum of reference chemical substances, a computational unit configured to perform an element-by-element division, a baseline removal filter

2. The light source according to claim 1, including one or all two of a light emitting diode or a lamp.

3. The optical sensor according to claim 1, comprising one or all two of a UV-visible spectrophotometer or a NIR spectrophotometer

4. The non-volatile memory according to claim 1, including a Flash memory or an EPROM memory

5. The computational unit according to claim 1, configured to perform an element by element division is either digital or analog

6. The peak overlapping discrimination procedure according to claim 1, including derivative computation, is applied to the optic detection system output

7. The system according to claim 1, configured to be transportable and integrates a battery.

8. The system according to claim 1, including one or all two of a wireless transmission element or a communication element using a hard-wired transmission configured to transmit information with a smart device or a computer.

9. The computation unit output according to claim 1, configured to be directly or indirectly reported to the user on a built-in display system.

10. A trace chemical detection method capable of value update, comprising the steps of: connecting to a distant data server configured to store reference chemical substances light spectra, said data server capable of updating or adding supplementary reference chemical substances light spectra to the ones already stored, comparing the light spectrum of each reference chemical substances with the light spectrum recorded by an optical detection system while applying broadband light to the analysed sample

11. The method of claim 10, wherein the light spectra comparison includes one or all two of an element-by-element division or a baseline removal filter

12. The method of claim 10, wherein the connection with the data server includes a wireless connection.
